(19) Europäisches Patentamt / European Patent Office / Office européen des brevets

(11) **EP 4 666 939 A1**

(12) **EUROPEAN PATENT APPLICATION**
published in accordance with Art. 153(4) EPC

(43) Date of publication:
24.12.2025 Bulletin 2025/52

(21) Application number: 24764177.2

(22) Date of filing: 27.02.2024

(51) International Patent Classification (IPC):
$A61B\ 5/0537^{(2021.01)}$    $A61B\ 5/00^{(2006.01)}$
$A61B\ 5/024^{(2006.01)}$    $A61B\ 5/01^{(2006.01)}$

(52) Cooperative Patent Classification (CPC):
A61B 5/00; A61B 5/01; A61B 5/024; A61B 5/0537

(86) International application number:
PCT/KR2024/002534

(87) International publication number:
WO 2024/181778 (06.09.2024 Gazette 2024/36)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.02.2023 KR 20230025830
27.03.2023 KR 20230039892

(71) Applicant: Samsung Electronics Co., Ltd.
Suwon-si, Gyeonggi-do 16677 (KR)

(72) Inventors:
• CHOI, Hyoungseon
Suwon-si Gyeonggi-do 16677 (KR)

• OH, Youngjae
Suwon-si Gyeonggi-do 16677 (KR)
• MIN, Jinhong
Suwon-si Gyeonggi-do 16677 (KR)
• JEON, Taehan
Suwon-si Gyeonggi-do 16677 (KR)
• KIM, Joonho
Suwon-si Gyeonggi-do 16677 (KR)
• KIM, Taeseon
Suwon-si Gyeonggi-do 16677 (KR)
• YOON, Seoyoung
Suwon-si Gyeonggi-do 16677 (KR)

(74) Representative: Gulde & Partner
Patent- und Rechtsanwaltskanzlei mbB
Berliner Freiheit 2
10785 Berlin (DE)

(54) **WEARABLE DEVICE FOR MONITORING BODY WATER, ELECTRONIC DEVICE, AND OPERATION METHOD THEREFOR**

(57) A wearable device according to an embodiment of the present disclosure may comprise: a communication circuit set to transmit or receive data or signals to or from an external electronic device; an output module; an electrical sensor that measures a first biological signal related to electrical properties; an optical sensor that measures a second biological signal related to optical properties; a temperature sensor that measures the temperature of skin; and at least one processor operatively connected to the communication circuit, the output module, the electrical sensor, the optical sensor, and the temperature sensor. The at least one processor may be configured to: acquire the first biological signal, the second biological signal, and a third biological signal related to the temperature from the electrical sensor, the optical sensor, and the temperature sensor, respectively; transmit first data related to the acquired first biological signal, second data related to the acquired second biological signal, and third data related to the third biological signal to the external electronic device via the communication circuit; receive data related to body water changes in the body from the external electronic device via the communication circuit, the body water changes being calculated on the basis of changes in the first data and the second data respectively corrected using the third data; and output feedback related to the body water changes to the output module on the basis of the received data related to body water changes.

FIG. 2A

**Description**

[Technical Field]

The disclosure relates to a wearable device, an electronic device, and an operating method thereof for monitoring a change in body water according to an embodiment.

[Background Art]

Maintaining body water is essential for basic human health such as blood circulation through smooth metabolism of the body, waste excretion, and body temperature regulation. In particular, when body water decreases by 2% or more, deterioration of cognitive ability and motor ability may occur and, when body water decreases by 6% or more, it may lead to death in severe cases.

Conventionally, to detect such body water, a dilution method using a radioactive isotope, a whole body composition analysis method utilizing a bioelectrical impedance analysis (BIA) method that measures and analyzes impedance in the body, or a sweat patch that collects and analyzes sweat from the skin has been used. However, these methods take a long time to measure and enable only temporary measurement, making it difficult to continuously monitor the state of body water. Further, the sweat patch is a method that may be utilized only during exercise when a large amount of sweat is discharged.

Further, in the case of an exercise state, which is a main cause of causing a change in body water, body temperature rises, causing an error in measuring the change in body water.

[Detailed Description of the Invention]

[Technical Solution]

A wearable device according to an embodiment of the disclosure may include a communication circuit configured to transmit or receive data or a signal with an external electronic device, an output module, an electrical sensor measuring a first biosignal related to an electrical characteristic, an optical sensor measuring a second biosignal related to an optical characteristic, a temperature sensor measuring a temperature of skin, and at least one processor operatively connected to the communication circuit, the output module, the electrical sensor, the optical sensor, and the temperature sensor. The at least one processor may be configured to obtain, from the electrical sensor, the optical sensor, and the temperature sensor, respectively, the first biosignal, the second biosignal, and a third biosignal related to the temperature. The at least one processor may be configured to transmit, through the communication circuit, first data related to the obtained first biosignal, second data related to the obtained second biosignal, and third data related to the third biosignal to the external electronic device. The at least one processor may be configured to receive, through the communication circuit, data related to a body water change of a body calculated based on changes in the first data and the second data respectively corrected by the third data from the external electronic device. The at least one processor may be configured to output feedback related to the body water change to the output module based on the received data related to the body water change.

A method of operating the wearable device according to an embodiment of the disclosure may include obtaining, from an electrical sensor, an optical sensor, and a temperature sensor, respectively, a first biosignal related to an electrical characteristic, a second biosignal related to an optical characteristic, and a third biosignal related to temperature. The method of operating the wearable device according to an embodiment may include transmitting, through a communication circuit, first data related to the obtained first biosignal, second data related to the obtained second biosignal, and third data related to the third biosignal to an external electronic device. The method of operating the wearable device according to an embodiment may include receiving, through the communication circuit, data related to a body water change of a body calculated based on changes in the first data and the second data respectively corrected by the third data from the external electronic device. The method of operating the wearable device according to an embodiment may include outputting feedback related to the body water change to an output module based on the received data related to the body water change.

A non-transitory, computer-readable storage medium storing one or more programs according to an embodiment of the disclosure may include obtaining, from an electrical sensor, an optical sensor, and a temperature sensor, respectively, a first biosignal related to an electrical characteristic, a second biosignal related to an optical characteristic, and a third biosignal related to temperature, based on execution of an application. The storage medium according to an embodiment may include transmitting, through a communication circuit, first data related to the obtained first biosignal, second data related to the obtained second biosignal, and third data related to the third biosignal to an external electronic device. The storage medium according to an embodiment may include receiving, through the communication circuit, data related to a body water change of a body calculated based on changes in the first data and the second data respectively corrected by the third data from the external electronic device. The storage medium according to an embodiment may include outputting feedback related to the body water change to an output module based on the received data related to the body water change.

An electronic device according to an embodiment of the disclosure may include a communication circuit configured to transmit or receive data or a signal with an external device, an output module, and at least one processor operatively connected to the communication circuit and the output module. The at least one processor may be configured to obtain first data related to a first biosignal corresponding to an electrical characteristic, second data related to a second biosignal corresponding to an optical characteristic, and third data corresponding to a temperature of skin. The at least one processor may be configured to correct the first data and the second data based on the third data. The at least one processor may be configured to calculate data related to a body water change of a body based on changes in the corrected first data and the corrected second data. The at least one processor may be configured to transmit, through the communication circuit, the calculated data related to the body water change to the external device, or output feedback to the output module based on the calculated data related to the body water change.

A method of operating an electronic device according to an embodiment of the disclosure may include obtaining first data related to a first biosignal corresponding to an electrical characteristic, second data related to a second biosignal corresponding to an optical characteristic, and third data related to a temperature of skin. The method of operating the electronic device according to an embodiment may include correcting the first data and the second data based on the third data. The method of operating the electronic device according to an embodiment may include calculating data related to a body water change of a body based on changes in the corrected first data and the corrected second data. The method of operating the electronic device according to an embodiment may include transmitting, through a communication circuit, the calculated data related to the body water change to an external device, or outputting feedback to an output module based on the calculated data related to the body water change.

A non-transitory, computer-readable storage medium storing one or more programs according to an embodiment of the disclosure may include obtaining first data related to a first biosignal corresponding to an electrical characteristic, second data related to a second biosignal corresponding to an optical characteristic, and third data related to a temperature of skin, based on execution of an application. The storage medium according to an embodiment may include correcting the first data and the second data based on the third data. The storage medium according to an embodiment may include calculating data related to a body water change of a body based on changes in the corrected first data and the corrected second data. The storage medium according to an embodiment may include transmitting, through a communication circuit, the calculated data related to the body water change to an external device, or outputting feedback to an output module based on the calculated data related to the body water change.

In a storage medium storing computer-readable instructions according to an embodiment of the disclosure, the instructions, when executed by at least one processor of a wearable device, may cause the wearable device to obtain the first biosignal, the second biosignal, and a third biosignal related to the temperature from an electrical sensor measuring a first biosignal related to an electrical characteristic, an optical sensor measuring a second biosignal related to an optical characteristic, and a temperature sensor measuring a temperature of skin, respectively.

The instructions may, when executed by at least one processor of the wearable device, cause the wearable device to transmit, through a communication circuit configured to transmit or receive data or a signal with an external electronic device, first data related to the obtained first biosignal, second data related to the obtained second biosignal, and third data related to the third biosignal to the external electronic device.

The instructions may, when executed by at least one processor of the wearable device, cause the wearable device to receive, through the communication circuit, data related to a body water change of a body calculated based on changes in the first data and the second data respectively corrected by the third data from the external electronic device.

The instructions may, when executed by at least one processor of the wearable device, cause the wearable device to output feedback related to the body water change to an output module based on the received data related to the body water change.

[Brief Description of Drawings]

FIG. 1 is a block diagram illustrating an electronic device in a network environment according to an embodiment of the disclosure;

FIG. 2A illustrates a block diagram illustrating a wearable device according to an embodiment of the disclosure;

FIG. 2B illustrates a connection state between a wearable device and an external electronic device according to an embodiment of the disclosure;

FIG. 3 is a flowchart regarding an operating method of a wearable device according to an embodiment of the disclosure;

FIG. 4 is a block diagram illustrating an electronic device according to an embodiment of the disclosure;

FIG. 5 is a flowchart regarding an operating method of an electronic device according to an embodiment of the disclosure;

FIG. 6 illustrates a change graph of body water calculated corresponding to whether temperature correction is performed according to an embodiment of the disclosure;

FIG. 7 illustrates a graph in which impedance of a plurality of frequencies changes according to a change in body temperature according to an embodiment of the disclosure;

FIG. 8A is a graph illustrating a change in a ratio of green light to infrared light of PPG corresponding to whether temperature change occurs according to an embodiment of the disclosure;

FIG. 8B is a graph illustrating a change in PPG DC level corresponding to whether temperature change occurs according to an embodiment of the disclosure;

FIG. 8C is a graph illustrating PPG waveforms corresponding to respective temperatures according to an embodiment of the disclosure;

FIG. 8D is a graph illustrating a change in PPG AC peak size corresponding to whether temperature change occurs according to an embodiment of the disclosure;

FIG. 9A illustrates a graph of a first weight designated according to a temperature of skin according to an embodiment of the disclosure;

FIG. 9B illustrates a graph of a second weight designated according to a temperature of skin according to an embodiment of the disclosure;

FIG. 10A is a graph illustrating a change in first data corresponding to respective circumstances of a user according to an embodiment of the disclosure;

FIG. 10B is a graph illustrating a change in second data corresponding to respective circumstances of a user according to an embodiment of the disclosure; and

FIG. 11 illustrates a second display of an electronic device providing visual feedback according to an embodiment of the disclosure.

[Mode for Carrying out the Invention]

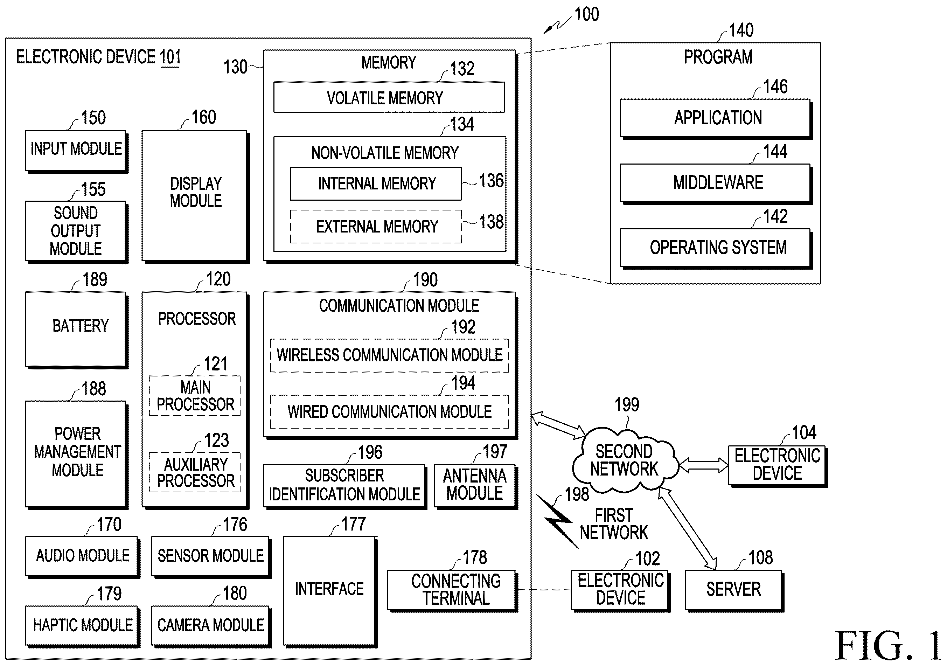

FIG. 1 is a block diagram illustrating an electronic device 101 in a network environment 100 according to an embodiment of the disclosure.

Referring to FIG. 1, the electronic device 101 in the network environment 100 may communicate with at least one of an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In an embodiment, at least one (e.g., the connecting terminal 178) of the components may be omitted from the electronic device 101, or one or more other components may be added in the electronic device 101. According to an embodiment, some (e.g., the sensor module 176, the camera module 180, or the antenna module 197) of the components may be integrated into a single component (e.g., the display module 160).

The processor 120 may execute, for example, software (e.g., the program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be configured to use lower power than the main processor 121 or to be specified for a designated function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. The artificial intelligence model may be generated via machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by other component (e.g., the processor 120) of the electronic device 101, from the outside (e.g., a user) of the electronic device 101. The input module 150 may include, for example, a microphone, a mouse, a keyboard, keys (e.g., buttons), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor configured to detect a touch, or a pressure sensor configured to measure the intensity of a force generated by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operation state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an accelerometer, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or motion) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device 104 via a first network 198 (e.g., a short-range communication network, such as Bluetooth™, wireless-fidelity (Wi-Fi) direct, or infrared data association (IrDA)) or a second network 199 (e.g., a long-range communication network, such as a legacy cellular network, a 5G network, a next-generation communication network, the Internet, or a computer network (e.g., local area network (LAN) or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device). According to an embodiment, the antenna module 197 may include one antenna including a radiator formed of a conductor or conductive pattern formed on a substrate (e.g., a printed circuit board (PCB)). According to an embodiment, the antenna module 197 may include a plurality of antennas (e.g., an antenna array). In this case, at least one antenna appropriate for a communication scheme used in a communication network, such as the first network 198 or the second network 199, may be selected from the plurality of antennas by, e.g., the communication module 190. The signal or the power may then be transmitted or received between the communication module 190 and the external electronic device via the selected at least one antenna. According to an embodiment, other parts (e.g., radio frequency integrated circuit (RFIC)) than the radiator may be further formed as part of the antenna module 197.

According to an embodiment, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. The external electronic devices 102 or 104 each may be a device of the same or a different type from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an Internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

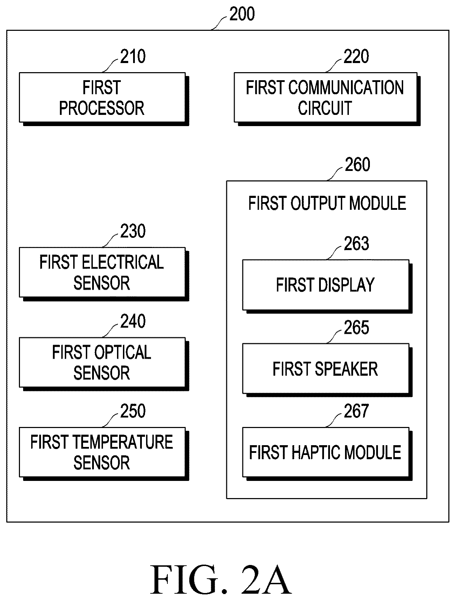

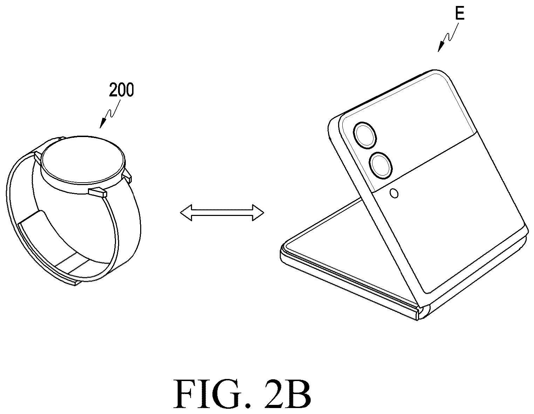

FIG. 2A illustrates a block diagram illustrating a wearable device 200 according to an embodiment of the disclosure. FIG. 2B illustrates a connection state between the wearable device 200 and an external electronic device E according to an embodiment of the disclosure.

Referring to FIG. 2A and FIG. 2B, a wearable device 200 (e.g., the electronic device 101 of FIG. 1) according to an embodiment may be a wearable type device that is bound to or mounted on a portion of a user's body. In an embodiment, the wearable device 200 may be a smart watch bound to the user's wrist, a head-mounted device (HMD) fixed to the user's head, or an ear-bud worn on the user's ear. In an embodiment, an external electronic device E (e.g., the external electronic device 102 of FIG. 1) may be a device that is communicatively connected to wirelessly transmit/receive signals or data with the wearable device 200. In an embodiment, the external electronic device E may be a portable communication device (e.g., a smartphone), a computer device, a portable multimedia device, or a portable medical device.

The wearable device 200 according to an embodiment may include a first processor 210 (e.g., the processor 120 of FIG. 1), a first communication circuit 220 (e.g., the communication module 190 of FIG. 1), a first electrical sensor 230, a first optical sensor 240, a first temperature sensor 250, and/or a first output module 260 (e.g., the sensor module 176 of FIG. 1). In an embodiment, the wearable device 200 may include only some of the components illustrated in FIG. 2A, or may additionally include components of the electronic device 101 illustrated in FIG. 1 that are not illustrated in FIG. 2A.

The first processor 210 according to an embodiment may perform an operation according to a command stored in first memory (not illustrated, e.g., the memory 130 of FIG. 1), or may perform an operation according to a command received from the external electronic device E. In an embodiment, the first memory (not illustrated) may store an application, data, and/or operation commands corresponding to operations performed by the first processor 210.

The first communication circuit 220 according to an embodiment may be wirelessly communicatively connected to the external electronic device E. For example, a communication method may be implemented in various ways such as Bluetooth, BLE, or Wi-Fi. In an embodiment, the first communication circuit 220 may set a connection state with the external electronic device E through a designated communication method, or transmit and/or receive signals or data with the external electronic device E.

The wearable device 200 according to an embodiment may obtain biometric data of the user through the first electrical sensor 230, the first optical sensor 240, and/or the first temperature sensor 250.

The first electrical sensor 230 according to an embodiment may obtain a biosignal related to electrical characteristics. In an embodiment, an electrode electrically connected to the first electrical sensor 230 may contact the user's body. In an embodiment, the wearable device 200 or the first processor 210 may estimate biometric data such as the user's heart rate, HRV, ECG, body temperature, water amount, sweat discharge amount, stress state, and breathing state using the biosignal obtained through the first electrical sensor 230.

For example, the first electrical sensor 230 may include an impedance sensor that measures data related to impedance corresponding to at least one frequency within a designated frequency band (e.g., 1 [kHz] to 3 [MHz] frequency band) range. For example, the first electrical sensor 230 may be an electrical dermal activity (EDA) sensor that measures galvanic skin response (GSR) or electrical dermal activity (EDA) related to electrical characteristics.

The first optical sensor 240 according to an embodiment may obtain a biosignal related to optical characteristics. In an embodiment, the first optical sensor 240 may obtain a biosignal related to optical characteristics by irradiating light onto the user's skin and obtaining light reflected by the skin. In an embodiment, the wearable device 200 or the first processor 210 may estimate biometric data such as the user's heart rate, respiratory rate, or blood pressure using the biosignal obtained through the first electrical sensor 230.

For example, the first optical sensor 240 may be a photoplethysmogram (PPG) type optical blood flow measurement sensor (photoplethysmography, PPG) that measures pulse waves using light.

In an embodiment, the wearable device 200 or the first processor 210 may estimate biometric data such as the user's heart rate, HRV, ECG, body temperature, water amount, sweat discharge amount, stress state, and breathing state using the biosignal obtained through the first optical sensor 240.

The first temperature sensor 250 according to an embodiment may measure the user's body temperature. For example, the first temperature sensor 250 may measure the temperature of skin while in contact with the user's skin. In an embodiment, the first temperature sensor 250 may measure the user's body temperature using optical or other methods.

The first output module 260 according to an embodiment may output visual, auditory, and/or tactile feedback to the user. In an embodiment, the first output module 260 may include a first display 263 providing visual feedback, a first speaker 265 providing auditory feedback, and/or a first haptic module 267 providing tactile feedback.

In an embodiment, the first output module 260 may output feedback related to body water change. For example, the first display 263 may display visual data related to body water change on a screen. For example, the first speaker 265 may generate sound related to body water change. For example, the first haptic module 267 may generate vibration related to body water change.

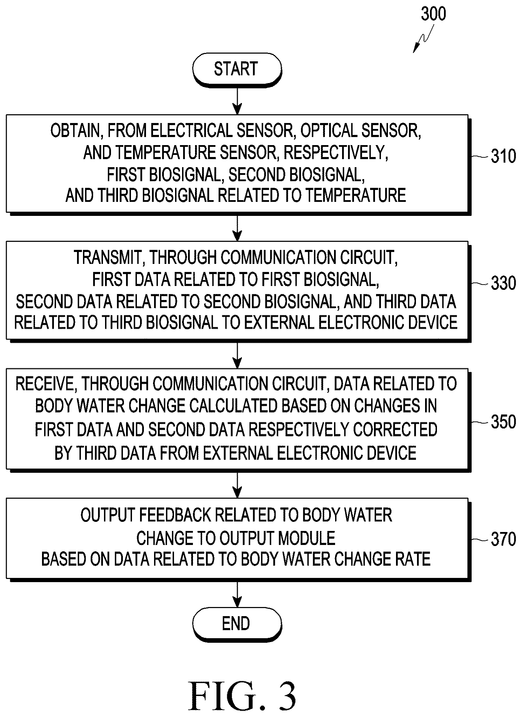

FIG. 3 is a flowchart 300 regarding an operating method of the wearable device 200 according to an embodiment of the disclosure.

Referring to FIG. 3, the wearable device (e.g., the wearable device 200 or the first processor 210 of FIG. 2A) according to an embodiment may obtain a first biosignal, a second biosignal, and a third biosignal related to temperature from an electrical sensor (e.g., the first electrical sensor 230 of FIG. 2A), an optical sensor (e.g., the first optical sensor 240 of FIG. 2A), and a temperature sensor (e.g., the first temperature sensor 250 of FIG. 2A), respectively, in operation 310.

In an embodiment, the wearable device 200 may obtain the first biosignal related to electrical characteristics from the electrical sensor 230. In an embodiment, the wearable device 200 may obtain the second biosignal related to optical characteristics from the optical sensor 240. In an embodiment, the wearable device 200 may obtain the third biosignal related to temperature from the temperature sensor 250, respectively.

In an embodiment, in operation 310, the wearable device 200 may obtain the first biosignal, the second biosignal, and/or the third biosignal simultaneously or sequentially, and the order of obtaining each signal may be changed in various ways.

The wearable device 200 according to an embodiment may transmit first data related to the obtained first biosignal, second data related to the obtained second biosignal, and third data related to the third biosignal to an external electronic device (e.g., the external electronic device E of FIG. 2B) through a communication circuit (e.g., the first communication circuit 220 of FIG. 2A) in operation 330.

In an embodiment, the wearable device 200 may set a communication connection corresponding to a designated communication method with the external electronic device E through the communication circuit 220. In an embodiment, the wearable device 200 may transmit the first data, the second data, and/or the third data to the external electronic device E in a designated communication method.

In an embodiment, the first data may be the first biosignal related to electrical characteristics obtained from the electrical sensor 230, and may be biometric data processed from the first biosignal. In an embodiment, the first data may be biometric data related to electrical characteristics such as impedance magnitude, impedance phase, resistance, reactance, or a ratio of high frequency to low frequency.

In an embodiment, the second data may be the second biosignal related to optical characteristics obtained from the optical sensor 240, and may be biometric data processed from the second biosignal. In an embodiment, the second data may be biometric data related to optical characteristics such as absorbance, PPG AC peak size, PPG DC level, PPG DC to AC, DC low wavelength to DC high wavelength, AC low wavelength to AC high wavelength.

In an embodiment, the third data may be the third biosignal related to temperature obtained from the temperature sensor 250, and may be biometric data processed from the third biosignal.

In an embodiment, in operation 330, the wearable device 200 may transmit the first data, the second data, and/or the third data simultaneously or sequentially, and the order of transmitting each signal may be changed in various ways.

The wearable device 200 according to an embodiment may receive data related to a body water change of a body calculated based on changes in the first data and the second data respectively corrected by the third data from the external electronic device E through the communication circuit 220 in operation 350.

The external electronic device E according to an embodiment may calculate the body water change of the body based on the first data, the second data, and the third data as described below. In an embodiment, the external electronic device E may correct the first data and the second data respectively using the third data received from the wearable device 200, and calculate the body water change based on changes in the respectively corrected first data and second data. In an embodiment, the external electronic device E may transmit data related to the calculated body water change of the body to the wearable device 200.

The wearable device 200 according to an embodiment may receive data related to the body water change of the body calculated based on the transmitted first data, second data, and third data from the external electronic device E in response to transmitting the first data, the second data, and the third data to the external electronic device E.

In an embodiment, the body water change of the body may mean a change based on a designated specific body water state. For example, the body water change of the body may be a change rate (e.g., +2% or -3%) for a specific body water state. For example, the specific body water state may be a body water state in a state satisfying a designated time or designated condition, an average of accumulated measurements, or a designated value.

The wearable device 200 according to an embodiment may output feedback related to the body water change to an output module (e.g., the first output module 260 of FIG. 2A) based on data related to the body water change received from the external electronic device E in operation 370.

The wearable device 200 according to an embodiment may display visual data related to the body water change on the first display 263. The wearable device 200 according to an embodiment may generate sound related to the body water change from the first speaker 265. The wearable device 200 according to an embodiment may generate vibration related to the body water change from the first haptic module 267.

The wearable device 200 according to an embodiment may output feedback corresponding to the body water change to the output module 260 in real-time based on data related to the body water change received from the external electronic device E. The wearable device 200 according to an embodiment may output feedback corresponding to the body water change to the output module 260 when the body water change received from the external electronic device E satisfies a designated condition.

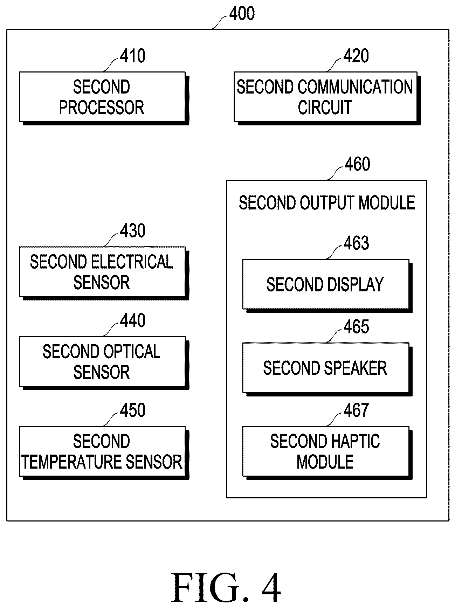

FIG. 4 is a block diagram illustrating an electronic device 400 according to an embodiment of the disclosure.

Referring to FIG. 4, an electronic device 400 (e.g., the electronic device 101 of FIG. 1) according to an embodiment may include a second processor 410 (e.g., the processor 120 of FIG. 1), a second communication circuit 420 (e.g., the communication module 190 of FIG. 1), a second electrical sensor 430, a second optical sensor 440, a second temperature sensor 450, and/or a second output module 460 (e.g., the sensor module 176 of FIG. 1). In an embodiment, the electronic device 400 may include only some of the components illustrated in FIG. 4, or may additionally include components of the electronic device 101 illustrated in FIG. 1 that are not illustrated in FIG. 4.

The electronic device 400 according to an embodiment may be a portable communication device (e.g., a smartphone), a computer device, a portable multimedia device, or a portable medical device. The electronic device 400 according to an embodiment may be a device that is communicatively connected to wirelessly transmit/receive signals or data with a wearable device (e.g., the wearable device 200 of FIG. 2A).

The electronic device 400 according to an embodiment may be a wearable type device such as a smart watch bound to the user's wrist that is bound to or mounted on a portion of the user's body, a head-mounted device (HMD) fixed to the user's head, or an ear-bud worn on the user's ear.

The second processor 410 according to an embodiment may perform an operation according to a command stored in second memory (not illustrated, e.g., the memory 130 of FIG. 1). In an embodiment, the second memory (not illustrated) may store an application, data, and/or operation commands. Operations executed by the second processor 210 and/or operations stored in the second memory (not illustrated) according to an embodiment is described below.

The second communication circuit 420 according to an embodiment may be wirelessly communicatively connected to the wearable device 200. For example, a communication method may be implemented in various ways such as Bluetooth, BLE, or Wi-Fi. In an embodiment, the first communication circuit 220 may set a connection state with the wearable device 200 through a designated communication method, or transmit and/or receive signals or data with the wearable device 200.

The electronic device 400 according to an embodiment may obtain biometric data of the user through the second electrical sensor 430, the first optical sensor 440, and/or the second temperature sensor 450. In an embodiment, the second electrical sensor 430, the second optical sensor 440, and/or the second temperature sensor 450 are components corresponding to the first electrical sensor 230, the first optical sensor 240, and/or the first temperature sensor 250, and descriptions related thereto are omitted.

The electronic device 400 according to an embodiment may receive first data related to a first biosignal corresponding to electrical characteristics, second data related to a second biosignal corresponding to optical characteristics, and third data corresponding to a temperature of skin through the second communication circuit 420. For example, the wearable device 200 may measure the first biosignal, the second biosignal, and/or the third biosignal, and transmit the first data, the second data, and/or the third data to the electronic device 400 through a communication circuit (e.g., the first communication circuit 220 of FIG. 2A).

The second output module 460 according to an embodiment may output visual, auditory, and/or tactile feedback to the user. In an embodiment, the second output module 460 may include a second display 463 providing visual feedback, a second speaker 465 providing auditory feedback, and/or a second haptic module 467 providing tactile feedback. In an embodiment, the second output module 460 is a component corresponding to the first output module (e.g., the first output module 260 of FIG. 2A), and descriptions related thereto are omitted.

The electronic device 400 according to an embodiment may transmit data related to body water change to the wearable device 200 through the second communication circuit 420. For example, the wearable device 200 may receive data related to body water change through a communication circuit (e.g., the first communication circuit 220 of FIG. 2A). For example, the wearable device 200 may output visual, auditory, and/or tactile feedback to an output module (e.g., the first output module 260 of FIG. 2A) based on the received data related to body water change.

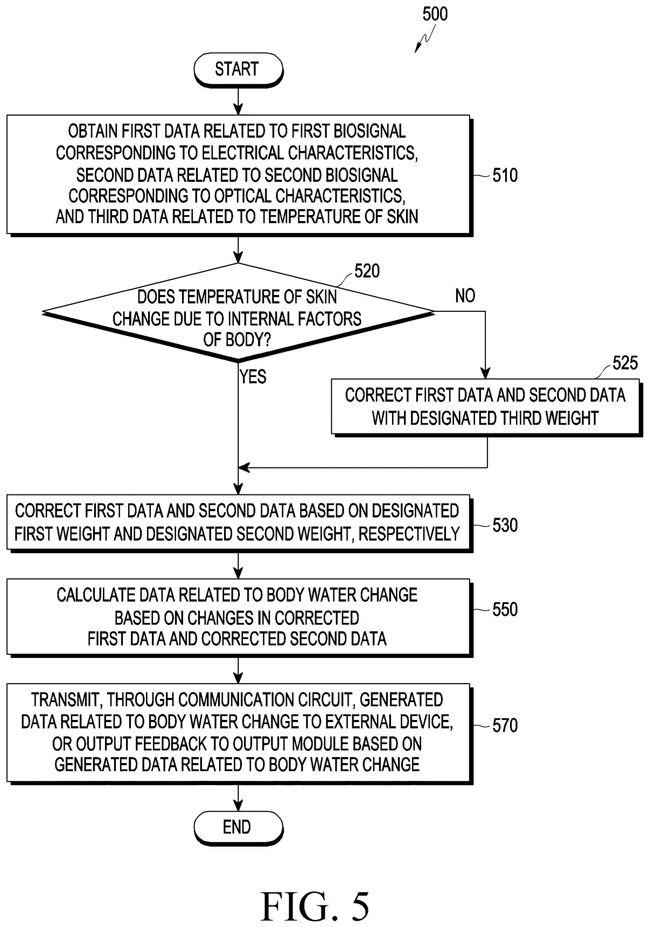

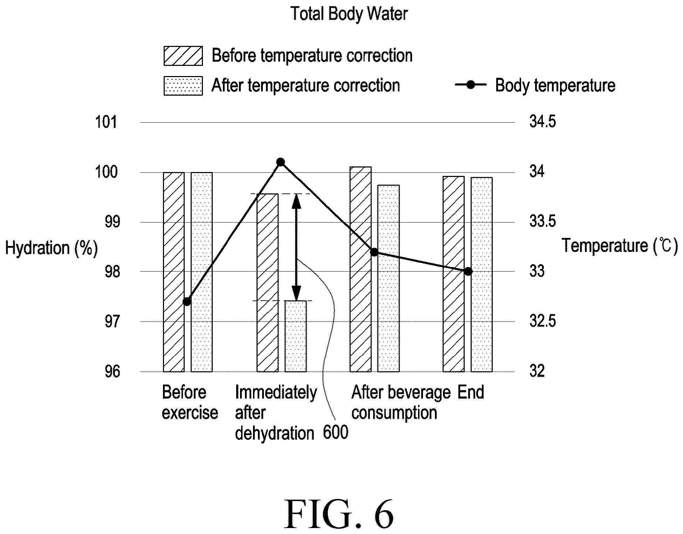

FIG. 5 is a flowchart 500 regarding an operating method of the electronic device 400 according to an embodiment of the disclosure. FIG. 6 illustrates a change graph of body water calculated corresponding to whether temperature correction is performed according to an embodiment of the disclosure.

Referring to FIG. 5, an electronic device (e.g., the electronic device 400 of FIG. 4) according to an embodiment may obtain first data related to a first biosignal corresponding to electrical characteristics, second data related to a second biosignal corresponding to optical characteristics, and third data corresponding to a temperature of skin.

The electronic device 400 according to an embodiment may obtain a first biosignal, a second biosignal, and a third biosignal related to temperature from an electrical sensor (e.g., the second electrical sensor 430 of FIG. 4), an optical sensor (e.g., the second optical sensor 440 of FIG. 4), and a temperature sensor (e.g., the second temperature sensor 450 of FIG. 4), respectively.

The electronic device 400 according to an embodiment may receive first data related to a first biosignal corresponding to electrical characteristics, second data related to a second biosignal corresponding to optical characteristics, and third data corresponding to a temperature of skin through the second communication circuit 420. The electronic device 400 according to an embodiment may receive the first data, the second data, and/or the third data from an external device (e.g., the wearable device 200 of FIG. 2A) through the second communication circuit 420.

The electronic device 400 according to an embodiment may obtain the first data, the second data, and/or the third data simultaneously or sequentially in operation 510, and the order of obtaining each signal may be changed in various ways.

The electronic device 400 according to an embodiment may identify whether the temperature of skin changes due to internal factors of the body in operation 520. For example, the temperature of skin may change due to internal factors of the body such as exercise or psychological state, and may also change due to external factors of the body according to changes in the external environment. The electronic device 400 according to an embodiment may identify whether it changes due to internal factors of the body or due to external factors of the body.

The electronic device 400 according to an embodiment may determine an exercise state of the user through heart rate (HR) or acceleration, and identify whether the temperature of skin changes due to internal factors of the body based on the determined exercise state.

The electronic device 400 according to an embodiment may determine whether the user is positioned outdoors based on location data (e.g., GPS signal), and obtain external environment data (e.g., temperature or humidity) corresponding to the user's location data. The electronic device 400 according to an embodiment may determine whether the user is positioned indoors based on location data (e.g., GPS signal), and obtain environment data (e.g., temperature or humidity) from an external device positioned indoors. The electronic device 400 according to an embodiment may include a separate sensor (not illustrated) that measures external environment data (e.g., temperature or humidity). The electronic device 400 according to an embodiment may identify whether the temperature of skin changes due to internal factors of the body based on the obtained external environment data.

The electronic device 400 according to an embodiment may obtain the user's core temperature from an external device (not illustrated). The electronic device 400 according to an embodiment may estimate the user's core temperature based on first data related to the first biosignal, second data related to the second biosignal, and/or third data corresponding to the temperature of skin. The electronic device 400 according to an embodiment may identify whether the temperature of skin changes due to internal factors of the body based on the user's core temperature.

The electronic device 400 according to an embodiment may identify whether the temperature of skin changes due to internal factors of the body after identifying whether the temperature of skin changes. For example, the electronic device 400 may identify whether the temperature of skin changes due to internal factors of the body when the temperature of skin changes by more than a designated temperature (e.g., 0.5 degrees).

The electronic device 400 according to an embodiment may correct the first data and the second data with a designated third weight in operation 525 based on a result of identifying whether the temperature of skin changes due to internal factors of the body.

The electronic device 400 according to an embodiment may not apply the designated third weight when the temperature of skin changes due to internal factors of the body (operation 520-Yes).

The electronic device 400 according to an embodiment may apply the designated third weight to the first data and the second data in operation 525 when the temperature of skin does not change due to internal factors of the body (operation 520-No).

In an embodiment, when the temperature of skin changes due to internal factors of the body, blood flow volume and/or physical property changes of blood may occur relatively largely. Conversely, when the temperature of skin changes due to external factors of the body, blood flow volume and/or physical property changes of blood may occur relatively small. For example, the designated third weight may be designated as a value smaller than 1.

The electronic device 400 according to an embodiment may correct the first data and the second data based on the third data in operation 530. The electronic device 400 according to an embodiment may correct the first data and the second data respectively based on the temperature of skin.

With further reference to FIG. 6, when the user performs exercise (e.g., internal factors of the body), body water may decrease compared to before exercise as the temperature of the body increases. However, a large error 600 occurs between changes in body water calculated respectively when temperature correction is performed and when temperature correction is not performed.

In particular, as illustrated in FIG. 6, when temperature correction is not performed, compared to when temperature correction is performed, changes in body water may not be properly reflected and errors may occur. The electronic device 400 according to an embodiment may quickly and accurately calculate changes in body water by detecting changes in body water based on biometric data corrected based on temperature.

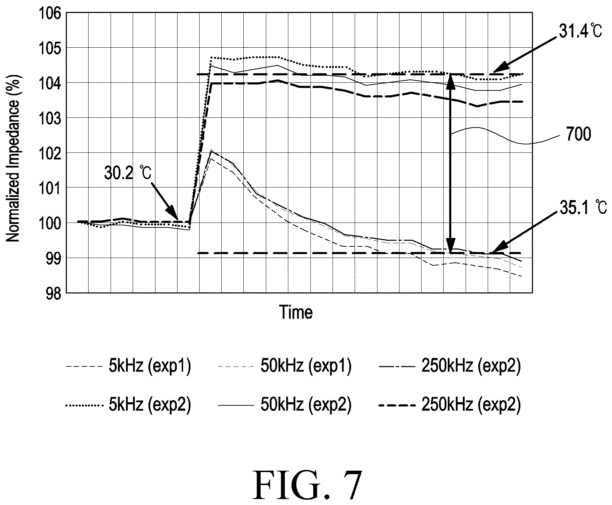

FIG. 7 illustrates a graph in which impedance of a plurality of frequencies changes according to a change in body temperature according to an embodiment of the disclosure.

With further reference to FIG. 7, a difference 700 may occur in the magnitude of impedance corresponding to electrical characteristics between when body temperature changes to a relatively high temperature (35.1°C) and when it changes to a relatively low temperature (31.4°C). A phenomenon in which impedance corresponding to a plurality of frequencies (5 kHz, 50 kHz, and 250 kHz) changes in response to changes in body temperature may be identified. This phenomenon may be caused by changes in blood flow volume according to temperature changes and/or changes in physical properties of blood according to temperature changes.

The impedance corresponding to each frequency in FIG. 7 has a difference 700 in the magnitude of impedance according to changes in body temperature as illustrated in [Table 1] below.

In an embodiment, the electronic device 400 may correct the measured impedance according to the temperature of skin. In an embodiment, the electronic device 400 may correct first data related to a first biosignal corresponding to electrical characteristics by applying a designated first weight. Here, the designated first weight is designated corresponding to the first data and may be designated to change according to the temperature of skin.

[Table 1]

| Frequency | High temperature (35.1°C) | Low temperature (31.4°C) | Impedance difference |
|---|---|---|---|
| 5 kHz | -1.53% | 4.37% | 5.90% |
| 50 kHz | -1.27% | 4.14% | 5.42% |
| 250 kHz | -1.10 % | 3.43% | 4.53% |

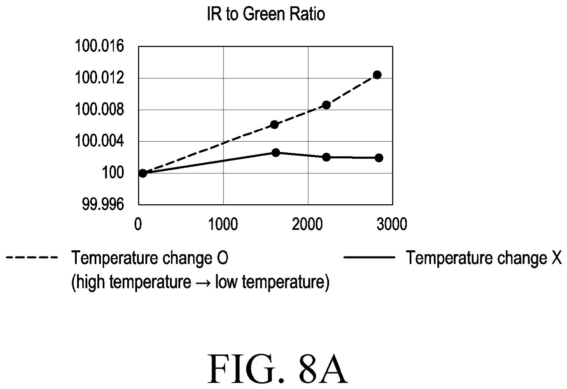

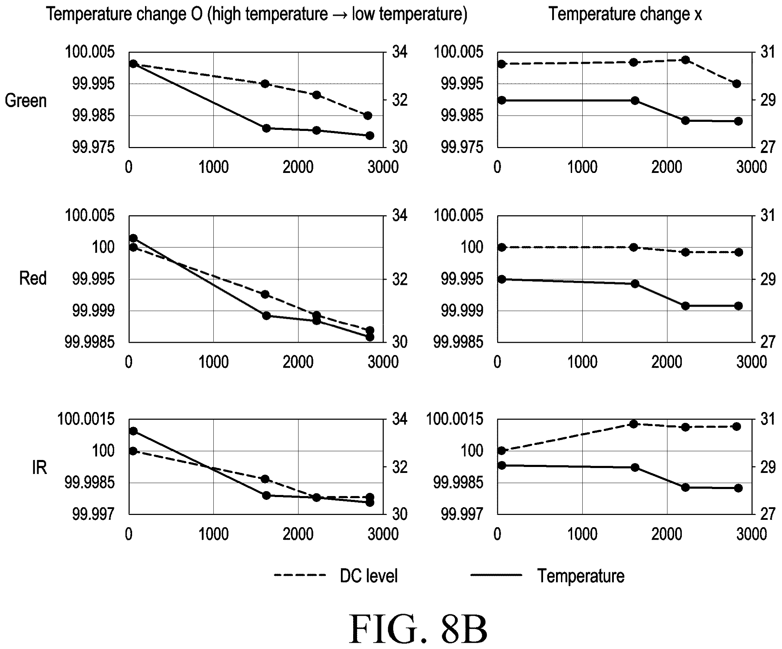

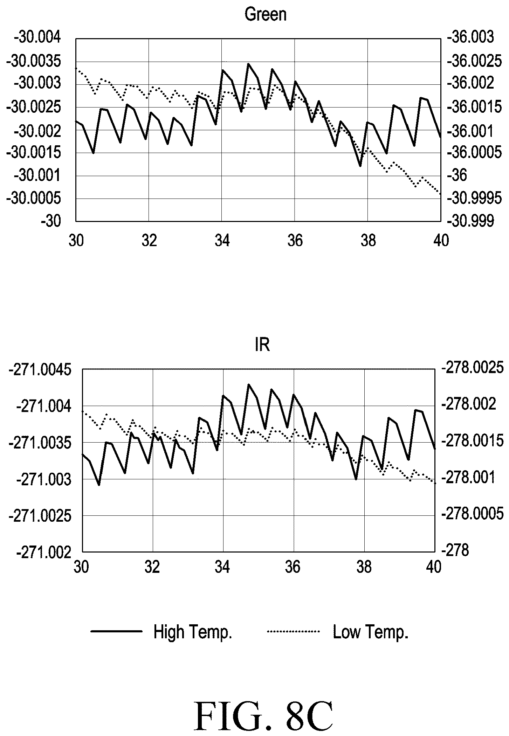

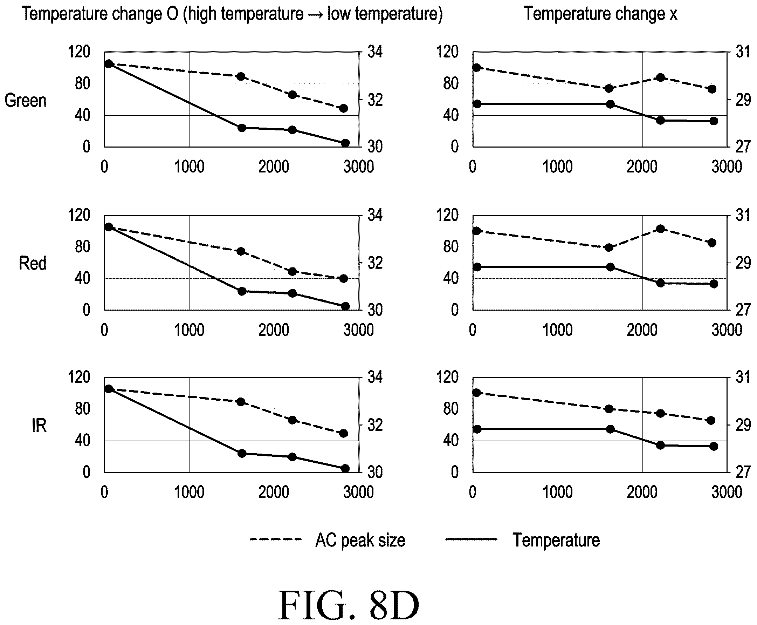

FIG. 8A is a graph illustrating a change in a ratio of green light to infrared light of PPG corresponding to whether temperature change occurs according to an embodiment of the disclosure. FIG. 8B is a graph illustrating a change in PPG DC level corresponding to whether temperature change occurs according to an embodiment of the disclosure. FIG. 8C is a graph illustrating PPG waveforms corresponding to respective temperatures according to an embodiment of the disclosure. FIG. 8D is a graph illustrating a change in PPG AC peak size corresponding to whether temperature change occurs according to an embodiment of the disclosure.

With further reference to FIG. 8A to FIG. 8D, compared to when no temperature change occurs, a phenomenon in which data related to PPG corresponding to optical characteristics changes differently when temperature change occurs may be identified.

Specifically, as illustrated in FIG. 8A, compared to when no temperature change occurs (temperature change X), when a temperature change from high temperature to low temperature occurs (temperature change O), a phenomenon in which the ratio of green light to infrared light (IR) gradually increases over time may be identified. For example, when a temperature change from high temperature to low temperature occurs (temperature change O), a phenomenon in which the ratio of green light to infrared light (IR) relatively increases may be identified.

As illustrated in FIG. 8B, when no temperature change occurs (temperature change X), the PPG DC level may be maintained almost constant. In contrast, when a temperature change from high temperature to low temperature occurs (temperature change O), a phenomenon in which the PPG DC level gradually decreases over time may be identified.

As illustrated in FIG. 8C, green light and infrared light (IR) may exhibit different PPG waveforms at relatively low temperature (Low Temp.) and relatively high temperature (High Temp). Specifically, in the case of green light, it may exhibit a relatively small change range at relatively low temperature (Low Temp.) and may exhibit an aspect in which the magnitude gradually decreases over time. Further, green light may exhibit a relatively large change range at relatively high temperature (High Temp) and may exhibit an aspect in which the magnitude is maintained over time.

In the case of infrared light (IR), it may exhibit a relatively small change range at relatively low temperature (Low Temp.) and may exhibit an aspect in which the magnitude gradually decreases over time. Further, infrared light (IR) may exhibit a relatively large change range at relatively high temperature (High Temp) and may exhibit an aspect in which the magnitude is maintained over time.

As illustrated in FIG. 8D, when no temperature change occurs (temperature change X), the PPG AC peak size may be maintained almost constant. In contrast, when a temperature change from high temperature to low temperature occurs (temperature change O), a phenomenon in which the PPG AC peak size gradually decreases over time may be identified.

In an embodiment, the electronic device 400 may correct the measured PPG or data related to PPG according to the temperature of skin. In an embodiment, the electronic device 400 may correct second data related to a second biosignal corresponding to optical characteristics by applying a designated second weight. Here, the designated second weight is designated corresponding to the second data and may be designated to change according to the temperature of skin.

In an embodiment, the designated first weight and second weight are designated corresponding to the first data and the second data respectively, and may be designated to increase or decrease respectively according to the temperature of skin. For example, the designated first weight and second weight may be mapped to a table or graph corresponding to changes in the temperature of skin.

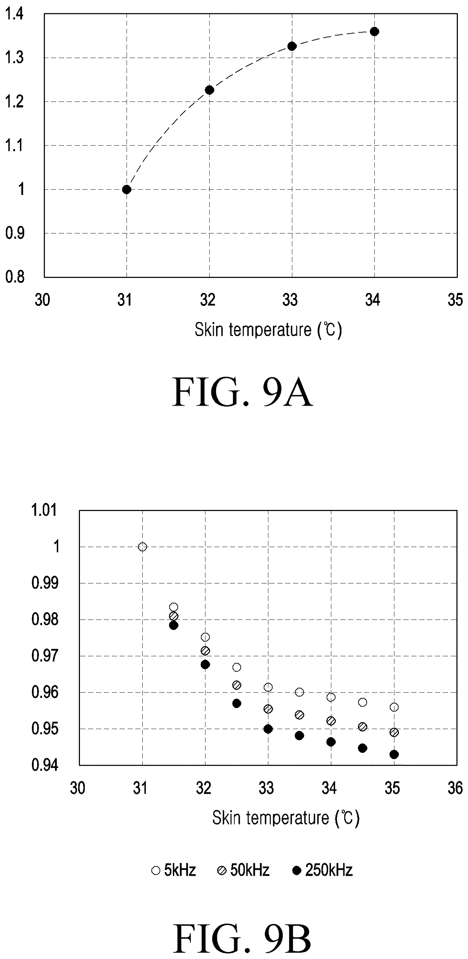

FIG. 9A illustrates a graph of a first weight designated according to a temperature of skin according to an embodiment of the disclosure. FIG. 9B illustrates a graph of a second weight designated according to a temperature of skin according to an embodiment of the disclosure.

[Table 2]

| Skin temperature (°C) | Designated first weight |
| --- | --- |
| ... | ... |
| 31 | 1 |
| 32 | 1.21 |
| 33 | 1.33 |
| 34 | 1.36 |
| ... | ... |

[Table 3]

| Skin temperature (°C) | Designated second weight | | |
| --- | --- | --- | --- |
| | 5 kHz | 50 kHz | 250 kHz |
| ... | ... | ... | ... |
| 31 | 1 | 1 | 1 |
| 31.5 | 0.9785 | 0.981 | 0.984 |
| 32 | 0.968 | 0.972 | 0.975 |
| 32.5 | 0.957 | 0.962 | 0.967 |
| ... | ... | ... | ... |
| 34 | 0.947 | 0.952 | 0.959 |
| 34.5 | 0.945 | 0.951 | 0.957 |
| 35 | 0.943 | 0.949 | 0.956 |
| ... | ... | ... | ... |

With further reference to FIG. 9A and Table 2, in an embodiment, the designated first weight may be designated to increase in magnitude based on an increase in the temperature of skin. For example, the designated first weight may be designated as 1 at a reference temperature (e.g., 31°C) and may be designated to be greater than 1 at a temperature increased from the reference temperature. In an embodiment, the designated first weight may be designated corresponding to first data related to a first biosignal corresponding to electrical characteristics respectively.

The electronic device 400 according to an embodiment may correct first data related to a first biosignal corresponding to electrical characteristics (e.g., magnitude of impedance) that decreases as the temperature of skin increases by applying the designated first weight that is designated to increase as the temperature of skin increases.

With further reference to FIG. 9B and Table 3, in an embodiment, the designated second weight may be designated to decrease in magnitude based on an increase in the temperature of skin. For example, the designated first weight may be designated as 1 at a reference temperature (e.g., 31°C) and may be designated to be smaller than 1 at a temperature increased from the reference temperature. In an embodiment, the designated second weight may be designated corresponding to second data related to a second biosignal corresponding to optical characteristics respectively.

The electronic device 400 according to an embodiment may correct second data related to a second biosignal corresponding to optical characteristics (e.g., ratio of green light to infrared light of PPG, PPG DC level, PPG AC peak size) that increases as the temperature of skin increases by applying the designated second weight that is designated to decrease as the temperature of skin increases.

Referring back to FIG. 5, the electronic device 400 according to an embodiment may calculate data related to a body water change of a body based on changes in the corrected first data and the corrected second data in operation 550.

The electronic device 400 according to an embodiment may calculate a change in the corrected first data. In an embodiment, the change in the corrected first data may be a current value (Corrected_Electrical_current) of the corrected first data compared to a previous value (Corrected_Electrical_previous) of the corrected first data as in the following equation (Equation 1). Alternatively, the change in the corrected first data may be calculated as a reciprocal of the current value (Corrected_Electrical_current) of the corrected first data compared to the previous value (Corrected_Electrical_previous) of the corrected first data as in the following equation (Equation 2).

[Equation 1]

$$ElectricalFeatureChange = CorrectedElectrical_{current}/CorrectedElectrical_{previous}$$

[Equation 2]

$$ElectricalFeatureChange = 1/(CorrectedElectrical_{current}/CorrectedElectrical_{previous})$$

The electronic device 400 according to an embodiment may calculate a change in the corrected second data. In an embodiment, the change in the corrected second data may be a current value (Corrected_Optical_current) of the corrected second data compared to a previous value (Corrected_Optical_previous) of the corrected second data as in the following equation (Equation 3). Alternatively, the change in the corrected first data may be calculated as a reciprocal of the current value of the corrected first data (Corrected_Optical_current) compared to the previous value (Corrected_Optical_previous) of the corrected second data as in the following equation (Equation 4).

[Equation 3]

$$OpticalFeatureChange = CorrectedOptical_{current}/CorrectedOptical_{previous}$$

[Equation 4]

$$OpticalFeatureChange = 1/(CorrectedOptical_{current}/CorrectedOptical_{previous})$$

The electronic device 400 according to an embodiment may calculate data related to body water change by applying a designated fifth weight and a designated sixth weight to the change in the corrected first data and the change in the corrected second data respectively.

In an embodiment, the electronic device 400 may calculate a body water change (Body Water Change) of the body based on a sum of a change in the corrected first data (Electrical Feature Change) to which the designated fifth weight (Weight 5) is applied and a change in the corrected second data (Optical Feature Change) to which the designated sixth weight (Weight 6) is applied as in the following equation (Equation 5).

[Equation 5]

$$BodyWaterChange = Weight5 \times ElectricalFeatureChange + Weight6 \times OpticalFeatureChange$$

In an embodiment, the sum of the designated fifth weight and the designated sixth weight may be maintained constant. For example, the sum of the designated fifth weight and the designated sixth weight may be maintained constant at 1.

In an embodiment, the designated fifth weight and the designated sixth weight may be changed based on a change in the temperature of skin. For example, the designated fifth weight and the designated sixth weight may be designated according to the temperature of skin as illustrated in Table 4 below.

[Table 4]

| Skin temperature (°C) | Designated fifth weight | Designated sixth weight |
|---|---|---|
| ... | ... | ... |
| 31 | 0.5 | 0.5 |
| 32 | 0.55 | 0.45 |
| 33 | 0.60 | 0.4 |
| 34 | 0.65 | 0.35 |
| ... | ... | ... |

In an embodiment, the designated fifth weight may be designated to gradually increase as the temperature of skin increases. In an embodiment, the designated sixth weight may be designated to gradually decrease as the temperature of skin increases.

For example, the temperature of skin often increases as the user performs exercise, and first data related to electrical characteristics may be relatively robust to motion artifact. In an embodiment, the magnitude of the designated fifth weight corresponding to first data related to electrical characteristics may be designated to be large.

Further, in the case of body water decrease (dehydration), since the reliability of first data related to electrical characteristics and second data related to optical characteristics is similar, the designated fifth weight and the designated sixth weight may be designated at similar levels.

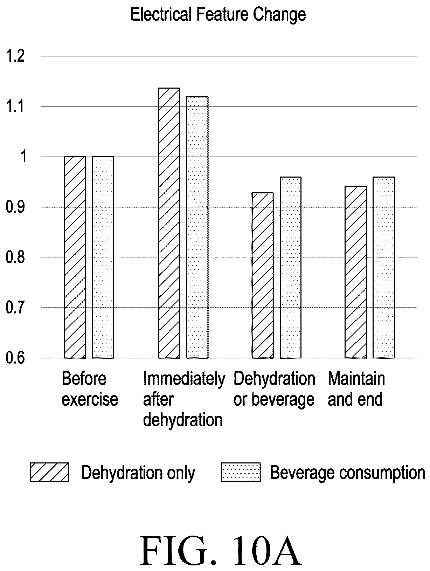

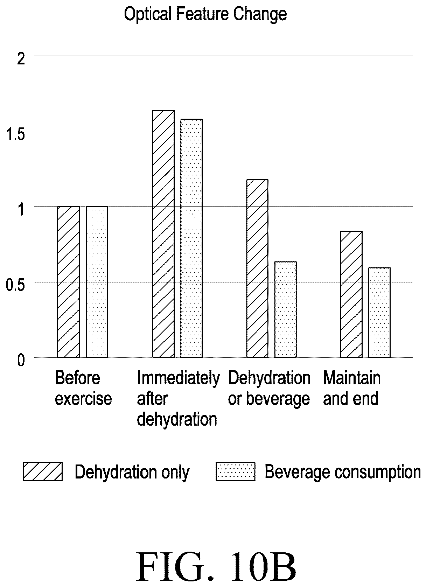

FIG. 10A is a graph illustrating a change in first data corresponding to respective circumstances of a user according to an embodiment of the disclosure. FIG. 10B is a graph illustrating a change in second data corresponding to respective circumstances of a user according to an embodiment of the disclosure.

With further reference to FIG. 10A, looking at the change in first data related to electrical characteristics when dehydration occurs due to user's exercise, almost no difference in the magnitude of change in first data occurs between when no beverage is consumed after dehydration (dehydration only) and when water is consumed after dehydration (beverage consumption). When water is consumed after dehydration (beverage consumption), it is expected that there is a difference in body water change of the body compared to when no beverage is consumed after dehydration (dehydration only). However, in the case of change in first data related to electrical characteristics, it may relatively less reflect the body water change of the body that changes in response to water consumption.

With further reference to FIG. 10B, looking at the change in second data related to optical characteristics when dehydration occurs due to user's exercise, a distinct difference in the magnitude of change in second data occurs between when no beverage is consumed after dehydration (dehydration only) and when water is consumed after dehydration (beverage consumption). In particular, after dehydration due to user's exercise, the change in second data exhibits a distinct difference when water is consumed (beverage consumption) compared to when no beverage is consumed (dehydration only). In the case of change in second data related to optical characteristics, it may relatively greatly reflect the body water change of the body that changes in response to water consumption.

In other words, in the case of body water increase (e.g., water re-consumption), the reliability of change in second data related to optical characteristics may be relatively high. In an embodiment, the magnitude of the designated sixth weight corresponding to second data related to optical characteristics may be designated to be relatively large.

In an embodiment, the designated fifth weight and the designated sixth weight may be designated according to body water change rate as illustrated in Table 5 below.

[Table 5]

| Body water change rate (%) | Designated fifth weight | Designated sixth weight |
|---|---|---|
| ... | ... | ... |
| +0.3% | 0.44 | 0.56 |
| +0.2% | 0.46 | 0.54 |
| +0.1% | 0.48 | 0.52 |
| 0 | 0.5 | 0.5 |
| -0.1% | 0.5 | 0.5 |
| ... | 0.5 | 0.5 |

In an embodiment, when there is no change in body water change rate or when body water decreases (e.g., dehydration), the designated fifth weight and the designated sixth weight may be designated to be maintained constant. In an embodiment, when body water increases (e.g., hydration), the designated fifth weight may be designated to increase and the designated sixth weight may be designated to decrease. In an embodiment, as the magnitude of the body water change rate increases, the designated fifth weight may be designated to gradually increase and the designated sixth weight may be designated to gradually decrease.

Referring back to FIG. 5, the electronic device 400 according to an embodiment may transmit data related to the generated body water change rate to an external device (e.g., the wearable device 200 of FIG. 2A) through a communication circuit in operation 570.

The electronic device 400 according to an embodiment may output feedback to an output module (e.g., the second output module 460 of FIG. 4) based on data related to the generated body water change rate in operation 570.

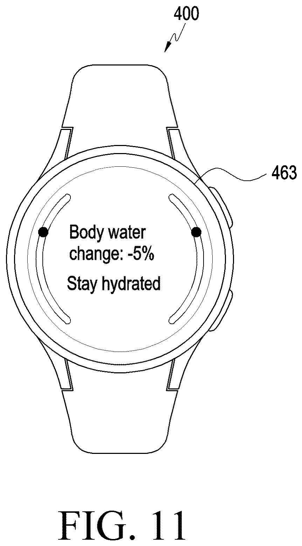

FIG. 11 illustrates a second display 463 of an electronic device 400 providing visual feedback according to an embodiment of the disclosure.

Referring to FIG. 11, the electronic device 400 according to an embodiment may output feedback to an output module (e.g., the second output module 460 of FIG. 4) based on data related to the generated body water change rate.

The electronic device 400 according to an embodiment may output visual, auditory, and/or tactile feedback to the second output module 460 to the user. In an embodiment, the electronic device 400 may output feedback related to the body water change rate based on data related to the generated body water change rate. In an embodiment, the electronic device 400 may provide visual feedback to the second display 463, provide auditory feedback to the second speaker 465, or provide tactile feedback to the second haptic module 467.

The electronic device 400 according to an embodiment may provide visual feedback to the second display 463 based on data related to the body water change rate as illustrated in FIG. 11. In an embodiment, the electronic device 400 may visually display data related to the generated body water change rate on the second display 463.

In an embodiment, the electronic device 400 may provide feedback to the second output module 460 when the body water change rate falls outside a designated range (e.g., -4% to +4% range) based on data related to the generated body water change rate.

A wearable device 200; 101 according to an embodiment of the disclosure may include a communication circuit 220; 190 configured to transmit or receive data or a signal with an external electronic device E, an output module 260, an electrical sensor 230 measuring a first biosignal related to an electrical characteristic, an optical sensor 240 measuring a second biosignal related to an optical characteristic, a temperature sensor 250 measuring a temperature of skin, and at least one processor 210; 120 operatively connected to the communication circuit 220; 190, the output module 260, the electrical sensor 230, the optical sensor 240, and the temperature sensor 250. The at least one processor 210; 120 may be configured to obtain, from the electrical sensor 230, the optical sensor 240, and the temperature sensor 250, respectively, the first biosignal, the second biosignal, and a third biosignal related to the temperature. The at least one processor 210; 120 may be configured to transmit, through the communication circuit 220; 190, first data related to the obtained first biosignal, second data related to the obtained second biosignal, and third data related to the third biosignal to the external electronic device E. The at least one processor 210; 120 may be configured to receive, through the communication circuit 220; 190, data related to a body water change of a body calculated based on changes in the first data and the second data respectively corrected by the third data from the external electronic device E. The at least one processor 210; 120 may be configured to output feedback related to the body water change to the output module 260 based on the received data related to the body water change.

In the wearable device 200; 101 according to an embodiment, the output module 260 may be configured to include at least one of a display 263 providing visual feedback, a speaker 265 providing auditory feedback, or a haptic module 267 providing tactile feedback.

In the wearable device 200; 101 according to an embodiment, the electrical sensor 230 may be measuring impedance of the skin corresponding to a specific frequency. The first data related to the first biosignal may include data related to at least one of a magnitude of impedance, a phase of impedance, a resistance, a reactance, or a ratio of impedance at different frequencies.

In the wearable device 200; 101 according to an embodiment, the optical sensor 240 may be configured to optically measure the second biosignal related to blood flow. The second data related to the second biosignal may include data related to at least one of absorbance, photoplethysmography PPG AC peak, PPG DC level, ratio between PPG DC and PPG AC, ratio of PPG DC at different frequencies, or ratio of PPG AC at different frequencies.

A method of operating the wearable device 200; 101 according to an embodiment of the disclosure may include obtaining 310, from an electrical sensor 230, an optical sensor 240, and a temperature sensor 250, respectively, a first biosignal related to an electrical characteristic, a second biosignal related to an optical characteristic, and a third biosignal related to temperature. The method of operating the wearable device 200; 101 according to an embodiment may include transmitting 330, through a communication circuit 220; 190, first data related to the obtained first biosignal, second data related to the obtained second biosignal, and third data related to the third biosignal to an external electronic device E. The method of operating the wearable device 200; 101 according to an embodiment may include receiving 350, through the communication circuit 220; 190, data related to a body water change of a body calculated based on changes in the first data and the second data respectively corrected by the third data from the external electronic device E. The method of operating the wearable device 200; 101 according to an embodiment may include outputting 370 feedback related to the body water change to an output module 260 based on the received data related to the body water change.

A non-transitory, computer-readable storage medium storing one or more programs according to an embodiment of the disclosure may include obtaining 310, from an electrical sensor 230, an optical sensor 240, and a temperature sensor 250, respectively, a first biosignal related to an electrical characteristic, a second biosignal related to an optical characteristic, and a third biosignal related to temperature, based on execution of an application. The storage medium according to an embodiment may include transmitting 330, through a communication circuit 220; 190, first data related to the obtained first biosignal, second data related to the obtained second biosignal, and third data related to the third biosignal to an external electronic device E. The storage medium according to an embodiment may include receiving 350, through the communication circuit 220; 190, data related to a body water change of a body calculated based on changes in the first data and the second data respectively corrected by the third data from the external electronic device E. The storage medium according to an embodiment may include outputting 370 feedback related to the body water change to an output module 260 based on the received data related to the body water change.

An electronic device 400; 101 according to an embodiment of the disclosure may include a communication circuit 420; 190 configured to transmit or receive data or a signal with an external device 200, an output module, and at least one processor 410; 120 operatively connected to the communication circuit 420; 190 and the output module 460. The at least one processor 410; 120 may be configured to obtain first data related to a first biosignal corresponding to an electrical characteristic, second data related to a second biosignal corresponding to an optical characteristic, and third data corresponding to a temperature of skin. The at least one processor 410; 120 may be configured to correct the first data and the second data based on the third data. The at least one processor 410; 120 may be configured to calculate data related to a body water change of a body based on changes in the corrected first data and the corrected second data. The at least one processor 410; 120 may be configured to transmit, through the communication circuit 420; 190, the calculated data related to the body water change to the external device 200, or output feedback to the output module 460 based on the calculated data related to the body water change.

In the electronic device 400; 101 according to an embodiment, the at least one processor 410; 120 may be configured to receive, through the communication circuit 420; 190, the first data, the second data, and the third data from the external device 200 as at least a portion of obtaining the first data, the second data, and the third data.

The electronic device 400; 101 according to an embodiment may further include an electrical sensor 430 measuring the first biosignal, an optical sensor 440 measuring the second biosignal, and a temperature sensor 450 measuring the temperature. The at least one processor 410; 120 may be configured to obtain, from the electrical sensor 430, the optical sensor 440, and the temperature sensor 450, respectively, the first data, the second data, and the third data, as at least a portion of obtaining the first data, the second data, and the third data.

In the electronic device 400; 101 according to an embodiment, the at least one processor 410; 120 may be configured to correct the first data and the second data based on a designated first weight and a designated second weight, respectively, as at least a portion of correcting the first data and the second data. The designated first weight may be designated to change according to the temperature of the skin corresponding to the first data. The designated second weight may be designated to change according to the temperature of the skin corresponding to the second data.

In the electronic device 400; 101 according to an embodiment, the designated first weight may be designated to increase in magnitude based on an increase in the temperature of the skin. The designated second weight may be designated to decrease in magnitude based on the increase in the temperature of the skin.

In the electronic device 400; 101 according to an embodiment, the at least one processor 410; 120 may be configured to identify whether the temperature of the skin changes due to an internal factor of the body. The at least one processor 410; 120 may be configured to correct the first data and the second data with a designated third weight based on the identified result.

In the electronic device 400; 101 according to an embodiment, the at least one processor 410; 120 may be configured to calculate the data related to the body water change based on a sum of a change in the corrected first data to which a designated fifth weight is applied and a change in the corrected second data to which a designated sixth weight is applied, as at least a portion of calculating the data related to the body water change of the body. A sum of the designated fifth weight and the designated sixth weight may be constant.

In the electronic device 400; 101 according to an embodiment, the designated fifth weight and the designated sixth weight may be changed based on a change in the temperature of the skin.

In the electronic device 400; 101 according to an embodiment, the designated fifth weight and the designated sixth weight may be changed based on the body water change.

A method of operating an electronic device 400; 101 according to an embodiment of the disclosure may include obtaining 510 first data related to a first biosignal corresponding to an electrical characteristic, second data related to a second biosignal corresponding to an optical characteristic, and third data related to a temperature of skin. The method of operating the electronic device 400; 101 according to an embodiment may include correcting 530 the first data and the second data based on the third data. The method of operating the electronic device 400; 101 according to an embodiment may include calculating 550 data related to a body water change of a body based on changes in the corrected first data and the corrected second data. The method of operating the electronic device 400; 101 according to an embodiment may include transmitting, through a communication circuit 420; 190, the calculated data related to the body water change to an external device 200, or outputting 570 feedback to an output module 460 based on the calculated data related to the body water change.

In the method of operating the electronic device 400; 101 according to an embodiment, the obtaining 510 the first data, the second data, and the third data may receive, through the communication circuit 420; 190, the first data, the second data, and the third data from the external device 200.

In the method of operating the electronic device 400; 101 according to an embodiment, the obtaining 510 the first data, the second data, and the third data may receive, from an electrical sensor 430 measuring the first biosignal, an optical sensor 440 measuring the second biosignal, and a temperature sensor 450 measuring the temperature, respectively, the first biosignal, the second biosignal, and the third biosignal.

In the method of operating the electronic device 400; 101 according to an embodiment, the correcting 530 the first data and the second data may correct the first data and the second data based on a designated first weight and a designated second weight, respectively. The designated first weight may be designated to change according to the temperature of the skin corresponding to the first data. The designated second weight may be designated to change according to the temperature of the skin corresponding to the second data.

The method of operating the electronic device 400; 101 according to an embodiment may further include identifying 520 whether the temperature of the skin changes due to an internal factor of the body. The method of operating the electronic device 400; 101 according to an embodiment may further include correcting 525 the first data and the second data with a designated third weight based on the identified result.

In the method of operating the electronic device 400; 101 according to an embodiment, the calculating 550 the data related to the body water change of the body may calculate the data related to the body water change based on a sum of a change in the corrected first data to which a designated fifth weight is applied and a change in the corrected second data to which a designated sixth weight is applied. A sum of the designated fifth weight and the designated sixth weight may be constant.

In the method of operating the electronic device 400; 101 according to an embodiment, the designated fifth weight and the designated sixth weight may be changed based on at least one of a change in the temperature of the skin or the body water change.

A non-transitory, computer-readable storage medium storing one or more programs according to an embodiment of the disclosure may include obtaining 510 first data related to a first biosignal corresponding to an electrical characteristic, second data related to a second biosignal corresponding to an optical characteristic, and third data related to a temperature of skin, based on execution of an application. The storage medium according to an embodiment may include correcting 530 the first data and the second data based on the third data. The storage medium according to an embodiment may include calculating 550 data related to a body water change of a body based on changes in the corrected first data and the corrected second data. The storage medium according to an embodiment may include transmitting, through a communication circuit 420; 190, the calculated data related to the body water change to an external device 200, or outputting 570 feedback to an output module 460 based on the calculated data related to the body water change.

In a storage medium storing computer-readable instructions according to an embodiment of the disclosure, wherein the instructions, when executed by at least one processor 210; 120 of a wearable device 200; 101, cause the wearable device 200; 101 to obtain, from an electrical sensor 230 measuring a first biosignal related to an electrical characteristic, an optical sensor 240 measuring a second biosignal related to an optical characteristic, and a temperature sensor 250 measuring a temperature of skin, respectively, the first biosignal, the second biosignal, and a third biosignal related to the temperature.

Through a communication circuit 220; 190 configured to transmit or receive data or a signal with an external electronic device E according to an embodiment of the disclosure, the instructions may cause to transmit first data related to the obtained first biosignal, second data related to the obtained second biosignal, and third data related to the third biosignal to the external electronic device E.

Through the communication circuit 220; 190 according to an embodiment of the disclosure, the instructions may cause to receive data related to a body water change of a body calculated based on changes in the first data and the second data respectively corrected by the third data from the external electronic device E.

Based on the received data related to the body water change according to an embodiment of the disclosure, the instructions may cause to output feedback related to the body water change to an output module 260.

The electronic device according to an embodiment may be one of various types of electronic devices. The electronic devices may include, for example, a portable communication device (e.g., a smart phone), a computer device, a portable multimedia device, a portable medical device, a camera, an electronic device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

An embodiment of the disclosure may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The storage medium readable by the machine may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program products may be traded as commodities between sellers and buyers. The computer program product may be distributed in the form of a machine-readable storage medium (e.g., compact disc read only memory (CD-ROM)), or be distributed (e.g., downloaded or uploaded) online via an application store (e.g., Play Store™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to an embodiment, each component (e.g., a module or a program) of the above-described components may include a single entity or multiple entities. Some of the plurality of entities may be separately disposed in different components. According to an embodiment, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable device (200; 101), comprising:

   a communication circuit (220; 190) configured to transmit or receive data or a signal with an external electronic device (E);
   an output module (260);
   an electrical sensor (230) configured to measure a first biosignal related to an electrical characteristic;
   an optical sensor (240) configured to measure a second biosignal related to an optical characteristic;

a temperature sensor (250) configured to measure a temperature of skin; and

at least one processor (210; 120) operatively connected to the communication circuit (220; 190), the output module (260), the electrical sensor (230), the optical sensor (240), and the temperature sensor (250), wherein the at least one processor (210; 120) is configured to:

obtain, from the electrical sensor (230), the optical sensor (240), and the temperature sensor (250), respectively, the first biosignal, the second biosignal, and a third biosignal related to the temperature;

transmit, through the communication circuit (220; 190), first data related to the obtained first biosignal, second data related to the obtained second biosignal, and third data related to the third biosignal to the external electronic device (E);

receive, through the communication circuit (220; 190), data related to a body water change of a body calculated based on changes in the first data and the second data respectively corrected by the third data from the external electronic device (E); and

output feedback related to the body water change to the output module (260) based on the received data related to the body water change.

2. The wearable device (200; 101) of claim 1, wherein the output module (260) includes at least one of a display (263) providing visual feedback, a speaker (265) providing auditory feedback, or a haptic module (267) providing tactile feedback.

3. The wearable device (200; 101) of claim 1 or 2,

wherein the electrical sensor (230) is configured to measure impedance of the skin corresponding to a specific frequency, and

wherein the first data related to the first biosignal includes data related to at least one of a magnitude of impedance, a phase of impedance, a resistance, a reactance, or a ratio of impedance at different frequencies.

4. The wearable device (200; 101) of any one of claims 1 to 3,

wherein the optical sensor (240) is configured to optically measure the second biosignal related to blood flow, and

wherein the second data related to the second biosignal includes data related to at least one of an absorbance, a photoplethysmography (PPG) AC peak, a PPG DC level, a ratio between PPG DC and PPG AC, a ratio of PPG DC at different frequencies, or a ratio of PPG AC at different frequencies.

5. An electronic device (400; 101), comprising:

a communication circuit (420; 190) configured to transmit or receive data or a signal with an external device (200);

an output module (460); and

at least one processor (410; 120) operatively connected to the communication circuit (420; 190) and the output module (460), wherein the at least one processor (410; 120) is configured to:

obtain first data related to a first biosignal corresponding to an electrical characteristic, second data related to a second biosignal corresponding to an optical characteristic, and third data corresponding to a temperature of skin;

correct the first data and the second data based on the third data;

calculate data related to a body water change of a body based on changes in the corrected first data and the corrected second data; and

transmit, through the communication circuit (420; 190), the calculated data related to the body water change to the external device (200), or output feedback to the output module (460) based on the calculated data related to the body water change.

6. The electronic device (400; 101) of claim 5, wherein the at least one processor (410; 120) is configured to receive, through the communication circuit (420; 190), the first data, the second data, and the third data from the external device (200) as at least a portion of obtaining the first data, the second data, and the third data.

7. The electronic device (400; 101) of claim 6, further comprising:

an electrical sensor (430) configured to measure the first biosignal;

an optical sensor (440) configured to measure the second biosignal; and
a temperature sensor (450) configured to measure the temperature, wherein the at least one processor (410; 120) is configured to obtain, from the electrical sensor (430), the optical sensor (440), and the temperature sensor (450), respectively, the first data, the second data, and the third data as at least a portion of obtaining the first data, the second data, and the third data.

8. The electronic device (400; 101) of any one of claims 5 to 7,

wherein the at least one processor (410; 120) is configured to correct the first data and the second data based on a designated first weight and a designated second weight, respectively, as at least a portion of correcting the first data and the second data,
wherein the designated first weight is designated to change according to the temperature of the skin corresponding to the first data, and
wherein the designated second weight is designated to change according to the temperature of the skin corresponding to the second data.

9. The electronic device (400; 101) of claim 8,

wherein the designated first weight is designated to increase in magnitude based on an increase in the temperature of the skin, and
wherein the designated second weight is designated to decrease in magnitude based on the increase in the temperature of the skin.

10. The electronic device (400; 101) of any one of claims 5 to 9, wherein the at least one processor (410; 120) is configured to:

identify whether the temperature of the skin changes due to an internal factor of the body; and
correct the first data and the second data with a designated third weight based on the identified result.

11. The electronic device (400; 101) of any one of claims 5 to 10,

wherein the at least one processor (410; 120) is configured to calculate the data related to the body water change based on a sum of a change in the corrected first data to which a designated fifth weight is applied and a change in the corrected second data to which a designated sixth weight is applied, as at least a portion of calculating the data related to the body water change, and
wherein a sum of the designated fifth weight and the designated sixth weight is constant.

12. The electronic device (400; 101) of claim 11, wherein the designated fifth weight and the designated sixth weight are changed based on a change in the temperature of the skin.

13. The electronic device (400; 101) of claim 11, wherein the designated fifth weight and the designated sixth weight are changed based on the body water change.

14. A method of operating an electronic device (400; 101), the method comprising:

obtaining (510) first data related to a first biosignal corresponding to an electrical characteristic, second data related to a second biosignal corresponding to an optical characteristic, and third data related to a temperature of skin;
correcting (530) the first data and the second data based on the third data;
calculating (550) data related to a body water change of a body based on changes in the corrected first data and the corrected second data; and
transmitting (570), through a communication circuit (420; 190), the calculated data related to the body water change to an external device (200), or outputting feedback to an output module (460) based on the calculated data related to the body water change.

15. A storage medium storing computer-readable instructions, wherein the instructions, when executed by at least one processor (210; 120) of a wearable device (200; 101), cause the wearable device (200; 101) to:

obtain, from an electrical sensor (230) measuring a first biosignal related to an electrical characteristic, an optical sensor (240) measuring a second biosignal related to an optical characteristic, and a temperature sensor (250) measuring a temperature of skin, respectively, the first biosignal, the second biosignal, and a third biosignal related to the temperature;

transmit first data related to the obtained first biosignal, second data related to the obtained second biosignal, and third data related to the third biosignal to an external electronic device (E) through a communication circuit (220; 190) configured to transmit or receive data or a signal with the external electronic device (E);

receive, through the communication circuit (220; 190), data related to a body water change of a body calculated based on changes in the first data and the second data respectively corrected by the third data from the external electronic device (E); and

output feedback related to the body water change to an output module (260) based on the received data related to the body water change.

FIG. 1

200

210
FIRST
PROCESSOR

220
FIRST COMMUNICATION
CIRCUIT

260
FIRST OUTPUT MODULE

230
FIRST ELECTRICAL
SENSOR

263
FIRST DISPLAY

240
FIRST OPTICAL
SENSOR

265
FIRST SPEAKER

250
FIRST TEMPERATURE
SENSOR

267
FIRST HAPTIC MODULE

# FIG. 2A

FIG. 2B

300

START

OBTAIN, FROM ELECTRICAL SENSOR, OPTICAL SENSOR,
AND TEMPERATURE SENSOR, RESPECTIVELY,
FIRST BIOSIGNAL, SECOND BIOSIGNAL,
AND THIRD BIOSIGNAL RELATED TO TEMPERATURE — 310

TRANSMIT, THROUGH COMMUNICATION CIRCUIT,
FIRST DATA RELATED TO FIRST BIOSIGNAL,
SECOND DATA RELATED TO SECOND BIOSIGNAL, AND THIRD DATA
RELATED TO THIRD BIOSIGNAL TO EXTERNAL ELECTRONIC DEVICE — 330

RECEIVE, THROUGH COMMUNICATION CIRCUIT, DATA RELATED TO
BODY WATER CHANGE CALCULATED BASED ON CHANGES IN
FIRST DATA AND SECOND DATA RESPECTIVELY CORRECTED
BY THIRD DATA FROM EXTERNAL ELECTRONIC DEVICE — 350

OUTPUT FEEDBACK RELATED TO BODY WATER
CHANGE TO OUTPUT MODULE
BASED ON DATA RELATED TO BODY WATER CHANGE RATE — 370

END

# FIG. 3

400

410

**SECOND PROCESSOR**

420

**SECOND COMMUNICATION CIRCUIT**

460

**SECOND OUTPUT MODULE**

430

**SECOND ELECTRICAL SENSOR**

463

**SECOND DISPLAY**

440

**SECOND OPTICAL SENSOR**

465

**SECOND SPEAKER**

450

**SECOND TEMPERATURE SENSOR**

467

**SECOND HAPTIC MODULE**

FIG. 4

500

START

OBTAIN FIRST DATA RELATED TO FIRST BIOSIGNAL
CORRESPONDING TO ELECTRICAL CHARACTERISTICS,
SECOND DATA RELATED TO SECOND BIOSIGNAL
CORRESPONDING TO OPTICAL CHARACTERISTICS,
AND THIRD DATA RELATED TO TEMPERATURE OF SKIN
— 510

DOES TEMPERATURE OF SKIN
CHANGE DUE TO INTERNAL FACTORS
OF BODY? — 520

NO

YES

CORRECT FIRST DATA AND SECOND DATA
WITH DESIGNATED THIRD WEIGHT — 525

CORRECT FIRST DATA AND SECOND DATA BASED ON DESIGNATED
FIRST WEIGHT AND DESIGNATED SECOND WEIGHT, RESPECTIVELY — 530

CALCULATE DATA RELATED TO BODY WATER CHANGE
BASED ON CHANGES IN CORRECTED
FIRST DATA AND CORRECTED SECOND DATA — 550

TRANSMIT, THROUGH COMMUNICATION CIRCUIT, GENERATED
DATA RELATED TO BODY WATER CHANGE TO EXTERNAL DEVICE,
OR OUTPUT FEEDBACK TO OUTPUT MODULE BASED ON
GENERATED DATA RELATED TO BODY WATER CHANGE — 570

END

FIG. 5

FIG. 6

FIG. 7

IR to Green Ratio

FIG. 8A

FIG. 8B

FIG. 8C

Temperature change O (high temperature → low temperature)

Temperature change x

Green

Red

IR

- - - - - AC peak size ——— Temperature

FIG. 8D

EP 4 666 939 A1

FIG. 9A

○ 5kHz    ⊘ 50kHz    ● 250kHz

FIG. 9B

Electrical Feature Change

FIG. 10A

Optical Feature Change

FIG. 10B

400

463

Body water
change: -5%

Stay hydrated

# FIG. 11

**EP 4 666 939 A1**

## INTERNATIONAL SEARCH REPORT

International application No.

**PCT/KR2024/002534**

| A. | CLASSIFICATION OF SUBJECT MATTER |
|---|---|

**A61B 5/0537**(2021.01)i; **A61B 5/00**(2006.01)i; **A61B 5/024**(2006.01)i; **A61B 5/01**(2006.01)i

According to International Patent Classification (IPC) or to both national classification and IPC

| B. | FIELDS SEARCHED |
|---|---|

Minimum documentation searched (classification system followed by classification symbols)

A61B 5/0537(2021.01); A61B 10/00(2006.01); A61B 5/00(2006.01); A61B 5/053(2006.01); A61B 5/318(2021.01); G16H 20/30(2018.01); G16H 20/60(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 웨어러블 (wearable), 센서 (sensor), 온도 (temperature), 광학 (optical), 체수분 (body water), 보정 (correction), 가중치 (weighting)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2022-0053682 A (QUALCOMM INCORPORATED) 29 April 2022 (2022-04-29) See paragraphs [0048], [0054], [0066], [0097] and [0098]; and claims 1 and 7. | 1-3,5-7,14,15 |
| A | | 8,9 |
| Y | WO 2016-030869 A1 (ECOLE POLYTECHNIQUE FEDERALE DE LAUSANNE (EPFL)) 03 March 2016 (2016-03-03) See abstract; page 9; and claim 2. | 1-3,5-7,14,15 |
| A | KR 10-0400870 B1 (KIM, Young Ae) 08 October 2003 (2003-10-08) See claims 1-3. | 1-3,5-9,14,15 |
| A | KR 10-2016-0055006 A (SAMSUNG ELECTRONICS CO., LTD.) 17 May 2016 (2016-05-17) See entire document. | 1-3,5-9,14,15 |

✓ Further documents are listed in the continuation of Box C.   ✓ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **31 May 2024** | **31 May 2024** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2024/002534** |

| C. | DOCUMENTS CONSIDERED TO BE RELEVANT | |
| --- | --- | --- |
| Category* | Citation of document, with indication, where appropriate, of the relevant passages | Relevant to claim No. |
| A | KR 10-2020-0042503 A (LVL TECHNOLOGIES, INC.) 23 April 2020 (2020-04-23)<br>See entire document. | 1-3,5-9,14,15 |

Form PCT/ISA/210 (second sheet) (July 2022)

## INTERNATIONAL SEARCH REPORT

International application No.

**PCT/KR2024/002534**

**Box No. II**   **Observations where certain claims were found unsearchable (Continuation of item 2 of first sheet)**

This international search report has not been established in respect of certain claims under Article 17(2)(a) for the following reasons:

1. ☐   Claims Nos.:
   because they relate to subject matter not required to be searched by this Authority, namely:

2. ☑   Claims Nos.: **12,13**
   because they relate to parts of the international application that do not comply with the prescribed requirements to such an extent that no meaningful international search can be carried out, specifically:

   Claims 12 and 13 refer to claim 11 which violates the manner of referring to dependent claims under PCT Rule 6.4(a).

3. ☑   Claims Nos.: **4,10,11**
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/KR2024/002534**

| Patent document cited in search report | | | Publication date (day/month/year) | Patent family member(s) | | | Publication date (day/month/year) |
|---|---|---|---|---|---|---|---|
| KR | 10-2022-0053682 | A | 29 April 2022 | CN | 105636504 | A | 01 June 2016 |
| | | | | CN | 105682541 | A | 15 June 2016 |
| | | | | CN | 113616170 | A | 09 November 2021 |
| | | | | CN | 114983432 | A | 02 September 2022 |
| | | | | CN | 114983433 | A | 02 September 2022 |
| | | | | EP | 3060099 | A1 | 31 August 2016 |
| | | | | EP | 3060099 | B1 | 22 November 2023 |
| | | | | EP | 3060100 | A1 | 31 August 2016 |
| | | | | EP | 4176799 | A2 | 10 May 2023 |
| | | | | EP | 4176799 | A3 | 28 June 2023 |
| | | | | EP | 4306053 | A2 | 17 January 2024 |
| | | | | EP | 4306053 | A3 | 10 April 2024 |
| | | | | JP | 2016-537063 | A | 01 December 2016 |
| | | | | JP | 2017-500069 | A | 05 January 2017 |
| | | | | JP | 2020-114488 | A | 30 July 2020 |
| | | | | JP | 2020-192398 | A | 03 December 2020 |
| | | | | JP | 2022-040338 | A | 10 March 2022 |
| | | | | JP | 2022-066442 | A | 28 April 2022 |
| | | | | JP | 2022-169787 | A | 09 November 2022 |
| | | | | JP | 2022-169788 | A | 09 November 2022 |
| | | | | JP | 2024-003131 | A | 11 January 2024 |
| | | | | JP | 6808486 | B2 | 06 January 2021 |
| | | | | JP | 7047032 | B2 | 04 April 2022 |
| | | | | KR | 10-2016-0075584 | A | 29 June 2016 |
| | | | | KR | 10-2016-0075585 | A | 29 June 2016 |
| | | | | KR | 10-2021-0125613 | A | 18 October 2021 |
| | | | | KR | 10-2022-0003676 | A | 10 January 2022 |
| | | | | KR | 10-2022-0035501 | A | 22 March 2022 |
| | | | | KR | 10-2344929 | B1 | 28 December 2021 |
| | | | | KR | 10-2348694 | B1 | 06 January 2022 |
| | | | | KR | 10-2371573 | B1 | 04 March 2022 |
| | | | | KR | 10-2388316 | B1 | 18 April 2022 |
| | | | | US | 10052035 | B2 | 21 August 2018 |
| | | | | US | 10478075 | B2 | 19 November 2019 |
| | | | | US | 10694957 | B2 | 30 June 2020 |
| | | | | US | 11918323 | B2 | 05 March 2024 |
| | | | | US | 11931132 | B2 | 19 March 2024 |
| | | | | US | 2015-0119654 | A1 | 30 April 2015 |
| | | | | US | 2015-0119725 | A1 | 30 April 2015 |
| | | | | US | 2019-0021606 | A1 | 24 January 2019 |
| | | | | US | 2020-0275842 | A1 | 03 September 2020 |
| | | | | US | 2021-0378521 | A1 | 09 December 2021 |
| | | | | WO | 2015-061166 | A1 | 30 April 2015 |
| | | | | WO | 2015-061783 | A1 | 30 April 2015 |
| WO | 2016-030869 | A1 | 03 March 2016 | None | | | |
| KR | 10-0400870 | B1 | 08 October 2003 | KR | 10-2002-0028493 | A | 17 April 2002 |
| KR | 10-2016-0055006 | A | 17 May 2016 | KR | 10-2335769 | B1 | 06 December 2021 |
| | | | | US | 10238312 | B2 | 26 March 2019 |
| | | | | US | 11627889 | B2 | 18 April 2023 |
| | | | | US | 2016-0128604 | A1 | 12 May 2016 |

Form PCT/ISA/210 (patent family annex) (July 2022)

**INTERNATIONAL SEARCH REPORT**
Information on patent family members

International application No.

**PCT/KR2024/002534**

| Patent document cited in search report | | | Publication date (day/month/year) | Patent family member(s) | | | Publication date (day/month/year) |
|---|---|---|---|---|---|---|---|
| | | | | US | 2019-0175055 | A1 | 13 June 2019 |
| | | | | US | 2023-0218192 | A1 | 13 July 2023 |
| KR | 10-2020-0042503 | A | 23 April 2020 | CA | 3073170 | A1 | 21 February 2019 |
| | | | | CN | 111132616 | A | 08 May 2020 |
| | | | | EP | 3668398 | A1 | 24 June 2020 |
| | | | | MX | 2020001846 | A | 24 November 2020 |
| | | | | US | 2020-0194106 | A1 | 18 June 2020 |
| | | | | WO | 2019-035027 | A1 | 21 February 2019 |

Form PCT/ISA/210 (patent family annex) (July 2022)